# EUROPEAN PATENT APPLICATION

(11) **EP 3 804 642 A1**
(43) Date of publication of application: **14.04.2021**
(21) Application number: 18919723.9
(22) Date of filing: 21.09.2018
(51) Int. Cl.: A61B 17/34, A61M 25/06

(54) **DILATOR**

(30) Priority: 24.05.2018 WO PCT/JP2018/019981
(71) Applicant: ASAHI INTECC CO., LTD., Seto-shi, Aichi 489-0071 (JP)
(72) Inventor: FUSEYA, Yukihiro, Nagoya-shi, Aichi 463-0024 (JP); TSUZUKU, Marina, Nagoya-shi, Aichi 463-0024 (JP)
(74) Representative: Prinz & Partner mbB
(86) International application number: PCT/JP2018/035087
(87) International publication number: WO 2019/225025

(57) **Abstract**

A dilator capable of easily and securely expanding a hole in an object is provided.

The dilator 1 includes a hollow shaft 11 and a spirally-arranged protruding portion 21, and the shaft 11 is either a shaft that has a tapered portion 111, a distal end portion 112, and a main body 113, or a shaft that does not have the distal end portion but has the tapered portion and the main body. When the shaft does not have the distal end portion, the spirally-arranged protruding portion is formed on outer peripheral surfaces of the tapered portion and the main body and has gaps between adjacent sections, and has a height of the spirally-arranged protruding portion smaller than a height of the spirally-arranged protruding portion, and when the shaft 11 has the distal end portion 112, the spirally-arranged protruding portion 21 is formed on outer peripheral surfaces s11 of the tapered portion 111, and the distal end portion 112 and/or the main body 113 and has gaps 214 between adjacent sections, and has a height t1 of the spirally-arranged protruding portion 211 smaller than a height t2 of the spirally-arranged protruding portion 212 and/or a height t3 of the spirally-arranged protruding portion 213.

## Description

### TECHNICAL FIELD

The present invention relates to a dilator.

### BACKGROUND ART

As an instrument for expanding a hole formed on a wall of an organ such as stomach and liver, or a constricted part in a body cavity such as bile duct and pancreatic duct, for example, a dilator is known. When using such a dilator, a distal end of the dilator is inserted into the aforementioned hole or the like, and a tapered portion of the dilator is pushed into the hole, so that the hole is expanded (e.g. see Patent Literature 1).

### CITATION LIST

### Patent Literature

Patent Literature 1: Japanese Unexamined Patent Application Publication No. 2008-11867

### SUMMARY OF INVENTION

### TECHNICAL PROBLEM

However, when using the conventional dilator as described above, a sufficient propulsion force cannot be ensured particularly on a tapered portion where an indentation resistance to a hole or a constricted part increases, and there is a concern that the hole and the like cannot be sufficiently expanded.

The present invention has been made on the basis of the aforementioned circumstances, and an object thereof is to provide a dilator that can easily and securely expand a hole, a constricted part formed on a wall of an organ or the like (hereinafter, also referred to as a "hole of object").

### SOLUTION TO PROBLEM

Some aspects of the present disclosure include
(1) a dilator including a hollow shaft and a spirally-arranged protruding portion, in which
   the shaft includes either
   a tapered portion having an outer diameter that is smaller at a distal end of the tapered portion than at a proximal end of the tapered portion,
   a distal end portion having a proximal end positioned on a distal end of the tapered portion and extending toward an axis-directional distal end side of the tapered portion, and
   a main body having a distal end positioned on a proximal end of the tapered portion and extending toward an axis-directional proximal end side of the tapered portion, or
   the tapered portion and the main body without the distal end portion, and
   when the shaft does not have the distal end portion, the spirally-arranged protruding portion is formed on each of outer peripheral surfaces of the tapered portion and the main body and has gaps between adjacent sections along an axial direction of the shaft, and
   the spirally-arranged protruding portion formed on the tapered portion has a height smaller than a height of the spirally-arranged protruding portion formed on the main body, and
   when the shaft has the distal end portion, the spirally-arranged protruding portion is formed on each of outer peripheral surfaces of the tapered portion, and the distal end portion and/or the main body and has gaps between adjacent sections along the axial direction of the shaft, and
   the spirally-arranged protruding portion formed on the tapered portion has a height smaller than heights of the spirally-arranged protruding portions formed on the distal end portion and/or the main body,
(2) the dilator according to (1), in which the shaft is composed of a first coil body including a wire wound around an axis of the shaft,
(3) the dilator according to (1) or (2), in which the spirally-arranged protruding portion is composed of a second coil body including a wire wound around on the outer peripheral surface of the shaft,
(4) the dilator according to (1), in which
   the shaft is composed of the first coil body including a wire wound around the axis of the shaft, and the spirally-arranged protruding portion is composed of the second coil body including a wire around on the outer peripheral surface of the shaft, and
   a winding direction of the wire on the first coil body and a winding direction of the wire on the second coil body are opposite to each other,
(5) the dilator according to (3) or (4), in which
   the wire of the second coil body wound around the outer peripheral surface of the tapered portion has a diameter smaller than a diameter of the wire of the second coil body wound around on the outer peripheral surface of the shaft excluding the tapered portion, and
(6) the dilator according to any one of (1) to (5), in which
   a pitch of the spirally-arranged protruding portion formed on the outer peripheral surface of the tapered portion is larger than a pitch of the spirally-arranged protruding portion formed on the outer peripheral surface of the shaft excluding the tapered portion.

Incidentally, the "distal end side" herein means a direction along the axial direction of the shaft in which the tapered portion is positioned with respect to the main body. In addition, the "proximal end side" means a direction along the axial direction of the dilator and opposite to the distal end side. In addition, the "distal end" refers to an end portion of the distal end side on any member or part, and the "proximal end" refers to an end portion of the proximal end side on any member or part. In addition, the "height of the protruding portion" means a size from the outer peripheral surface of the shaft where the spirally-arranged protruding portion is positioned to the outer peripheral end of the spirally-arranged protruding portion when viewed from the front in the axial direction of the shaft, and specifically, for example, means each of heights t1 to t3 of the spirally-arranged protruding portion in each figure herein.

### ADVANTAGEOUS EFFECTS OF INVENTION

The present invention can provide a dilator capable of easily and securely expanding a hole in an object.

### BRIEF DESCRIPTION OF DRAWINGS

FIG. 1 is a partially cut-out schematic side view illustrating the whole of the first embodiment of the present invention.
FIG. 2 is a partially cut-out schematic enlarged side view of a main part illustrating a first variation of FIG. 1.
FIG. 3 is a partially cut-out schematic enlarged side view of a main part illustrating a second variation of FIG. 1.
FIG. 4 is a partially cut-out schematic enlarged side view of a main part illustrating a third variation of FIG. 1.
FIG. 5 is a partially cut-out schematic enlarged side view of a main part illustrating a fourth variation of FIG. 1.
FIG. 6 is a schematic enlarged side view of a main part illustrating the second embodiment of the disclosed embodiments.
FIG. 7 is a partially cut-out schematic enlarged side view of a main part illustrating the third embodiment of the disclosed embodiments.
FIG. 8 is a schematic side view illustrating the whole of the fourth embodiment of the disclosed embodiments.
FIG. 9 is a schematic enlarged side view of a main part illustrating a first variation of FIG. 8.
FIG. 10 is a schematic enlarged side view of a main part illustrating a second variation of FIG. 8.
FIG. 11 is a schematic enlarged side view of a main part illustrating a third variation of FIG. 8.
FIG. 12 is a schematic enlarged side view of a main part illustrating a fourth variation of FIG. 8.
FIG. 13 is a schematic side view illustrating the whole of a fifth variation of FIG. 8.
FIG. 14 is a partially cut-out schematic side view illustrating the whole of the fifth embodiment of the disclosed embodiments.
FIG. 15 is a schematic side view illustrating the whole of the sixth embodiment of the disclosed embodiments.
FIG. 16 is a schematic enlarged side view of a main part illustrating the seventh embodiment of the disclosed embodiments.
FIG. 17 is a partially cut-out schematic enlarged side view of a main part illustrating another embodiment of the disclosed embodiments.
FIG. 18 is a partially cut-out schematic enlarged side view of a main part illustrating another embodiment of the disclosed embodiments.
FIG. 19 is a partially cut-out schematic enlarged side view of a main part illustrating another embodiment of the disclosed embodiments.
FIG. 20 is a partially cut-out schematic enlarged side view of a main part illustrating another embodiment of the disclosed embodiments.
FIG. 21 is a partially cut-out schematic enlarged side view of a main part illustrating another embodiment of the disclosed embodiments.
FIG. 22 is a partially cut-out schematic enlarged side view of a main part illustrating another embodiment of the disclosed embodiments.
FIG. 23 is a partially cut-out schematic enlarged side view of a main part illustrating another embodiment of the disclosed embodiments.

### DESCRIPTION OF EMBODIMENTS

The dilator includes a hollow shaft and a spirally-arranged protruding portion, and is characterized in that the shaft includes either a tapered portion having an outer diameter that is smaller at a distal end of the tapered portion than at a proximal end of the tapered portion, a distal end portion having a proximal end positioned on a distal end of the tapered portion and extending toward an axis-directional distal end side of the tapered portion, and a main body having a distal end positioned on a proximal end of the tapered portion and extending toward an axis-directional proximal end side of the tapered portion, or the tapered portion and the main body without the distal end portion, and if the shaft does not have the distal end portion, the spirally-arranged protruding portion is formed on each of outer peripheral surfaces of the tapered portion and the main body and has gaps between adjacent sections along an axial direction of the shaft, and the spirally-arranged protruding portion formed on the tapered portion has a height smaller than a height of the spirally-arranged protruding portion formed on the main body, and if the shaft has the distal end portion, the spirally-arranged protruding portion is formed on each of outer peripheral surfaces of the tapered portion, and the distal end portion and/or the main body and has gaps between adjacent sections along the axial direction of the shaft, and the spirally-arranged protruding portion formed on the tapered portion has a height smaller than heights of the spirally-arranged protruding portions formed on the distal end portion and/or the main body.

Hereinafter, the first to seventh embodiments of the present invention will be explained with reference to the drawings, but the disclosed embodiments are not limited to the embodiments illustrated in the FIGs. In addition, sizes of the dilator illustrated in each figure are described to make it easier to understand the contents of the embodiments, and do not correspond to the actual sizes.

### [First Embodiment]

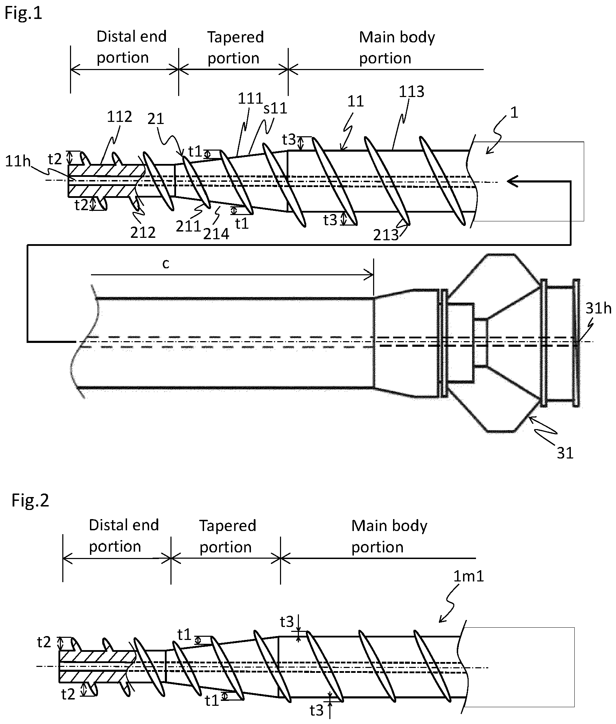

FIG. 1 is a partially cut-out schematic side view illustrating the whole of the first embodiment of the present invention. As illustrated in FIG. 1, a dilator 1 is roughly composed of a shaft 11, a spirally-arranged protruding portion 21, and a base portion 31.

The shaft 11 is a hollow member having a through-hole 11h. The through-hole 11h is e.g. a through-hole through which a guide wire (not illustrated) or the like is passed, and is composed of a continuous space that connects a distal end and a proximal end of the shaft 11 such that the guide wire or the like can freely pass. This shaft 11 has a tapered portion 111, a distal end portion 112, and a main body 113.

The tapered portion 111 is a part whose outer diameter at its distal end is smaller than at its proximal end. Specifically, the tapered portion 111 is connected to a distal end of the main body 113 described below, extends from this distal end toward an distal end side, and has a shape tapered toward the distal end side.

The distal end portion 112 is a part having a proximal end positioned on the distal end of the tapered portion 111 and extending toward the axis-directional distal end side of the tapered portion 111. Specifically, the distal end portion 112 has e.g. a proximal end continuous with the distal end of the tapered portion 111 and has a substantially constant outer diameter from the distal end to the proximal end. Incidentally, the distal end portion 112 can be formed integrally with or separately from the tapered portion 111. In the present embodiment, the distal end portion 112 and the tapered portion 111 are integrally formed by casting or the like.

The main body 113 is a part having the distal end positioned on the proximal end of the tapered portion 111 and extending toward the axis-directional proximal end side of the tapered portion 111. Specifically, in the main body 113, for example, the distal end is continuous with the proximal end of the tapered portion 111, and the proximal end is connected to the base portion 31 described below. The main body 113 has a substantially constant outer diameter from the distal end to the proximal end. Incidentally, the main body 113 can be formed integrally with or separately from the tapered portion 111. In the present embodiment, the main body 113 and the tapered portion 111 are integrally formed by casting or the like.

Preferably, materials constituting the distal end portion 112, the tapered portion 111, and the main body 113 described above have antithrombogenicity, flexibility, and biocompatibility because they are inserted into a body cavity. For example, a resin material such as a polyamide resin, a polyolefin resin, a polyester resin, a polyurethane resin, a silicone resin, and a fluororesin; a metal material such as a stainless steel and a superelastic alloy (nickel-titanium alloy); or the like can be adopted.

The distal end portion 112, the tapered portion 111, and the main body 113 may have various coating films (not illustrated) on sides of their surfaces (outer peripheral surfaces). Examples of the coating film include protective films (plating film as a typical example) for protecting the surfaces of the distal end portion 112, the tapered portion 111, and the main body 113.

The spirally-arranged protruding portion 21 is formed on each of the outer peripheral surfaces of the tapered portion, and the distal end portion and/or the main body, and has gaps between adjacent sections along the axial direction of the shaft. The spirally-arranged protruding portion formed on the tapered portion has a height smaller than a height of the spirally-arranged protruding portion formed on the distal end portion and/or the main body. Specifically, the spirally-arranged protruding portion 21 projects from an outer peripheral surface s11 of the shaft 11 toward an outside in a radial direction (outermost surface and outermost portion of the dilator 1), and is arranged such that the adjacent sections of the spirally-arranged protruding portion 21 are axially distant (with gaps 214).

In the present embodiment, the spirally-arranged protruding portion 21 is formed on the outer peripheral surface s11 of the shaft 11 (composed of the tapered portion 111, the distal end portion 112, and the main body 113), and formed such that a height t1 of a spirally-arranged protruding portion 211 formed on the tapered portion 111 is smaller than heights t2 and t3 of spirally-arranged protruding portions 212 and 213 formed on the distal end portion 112 and the main body 113 (t2> t1, and t1<t3).

Incidentally, the spirally-arranged protruding portion 21 can be formed as a single-thread or multi-thread projecting portion that is continuous or intermittent in the axial direction. In addition, the spirally-arranged protruding portion 21 may be formed either integrally with or separately from the shaft 11.

In the present embodiment, the spirally-arranged protruding portion 21 is formed as a continuous single-thread projecting portion, and the spirally-arranged protruding portion 21 is formed integrally with the distal end portion 112, the tapered portion 111, and the main body 113 by casting or the like.

Herein, it is preferable that the spirally-arranged protruding portion 21 does not constitute a blade (not have a shape that cuts a living tissue). That is, the spirally-arranged protruding portion 21 preferably has a transverse sectional shape (transverse section orthogonal to the spiral direction of the spirally-arranged protruding portion 21) in which a radial outer end portion of the shaft 11 is not an acute-angled corner. Examples of such an end portion include an end portion composed of an obtuse-angled corner, an end portion composed of a corner having a shape including a curved line (e.g. a curved line containing a part of a circle or an ellipse), and the like. Thereby, the dilator 1 can expand a hole on an object without damaging the living tissue on an inner face of the hole.

A material constituting the spirally-arranged protruding portion 21 is not particularly limited as long as the effect of the disclosed embodiments is not impaired. For example, a metal material such as a stainless steel and a superelastic alloy (nickel-titanium alloy); a biocompatible resin material such as a polyamide resin and a fluororesin; or the like can be used.

The base portion 31 is a part where an operator pushes the dilator 1 into a body and rotates the dilator 1. This base portion 31 has the distal end connected to the proximal end of the main body 113 and has a through-hole 31h communicating with the through-hole 11h of the shaft 11. During the operation, a guide wire or the like is inserted into the through-hole 11h through the through-hole 31h of the base portion.

Ordinarily, axial lengths of the respective parts of the dilator 1 are 1,600 to 2,500 mm in the whole shaft 11, 0 to 100 mm in the distal end portion 112, and 5 to 100 mm in the tapered portion 111. Ordinarily, outer diameters of the respective parts of the shaft 11 are 0.8 to 3.0 mm in the distal end portion 112 and the distal end of the tapered portion 111, and 1.4 to 5.0 mm in the proximal end of the tapered portion 111 and the main body 113. Ordinarily, an inner diameter of the through-hole 11h of the shaft 11 is 0.4 to 1.0 mm. Ordinarily, as for the heights of the spirally-arranged protruding portion 21, the height t2 in the distal end portion 112 is 0.125 to 0.5 mm, the height t1 in the tapered portion 111 is 0.1 to 0.4 mm, and the height t3 in the main body 113 is 0.125 to 0.5 mm. Preferably, a ratio of the height t1 of the spirally-arranged protruding portion 211 to the height t2 or t3 of the spirally-arranged protruding portion 212 or 213 (t1/t2, or t1/t3) is 1/5 to less than 1.

The axial lengths of the respective parts of the dilator 1 according to the present embodiment are 2,000 mm in the whole shaft 11, 10 mm in the distal end portion 112, and 30 mm in the tapered portion 111. The outer diameters of the respective parts of the shaft 11 are 1.84 mm in the distal end portion 112 and the distal end of the tapered portion 111, and 2.64 mm in the proximal end of the tapered portion 111 and the main body 113. The inner diameter of the through-hole 11h of the shaft 11 is 0.7 mm. In the spirally-arranged protruding portion 21, the height t1 is 0.2 mm, the height t2 is 0.3 mm, the height t3 is 0.3 mm. As for the ratio of the height of the spirally-arranged protruding portion 211 to the height of the spirally-arranged protruding portion 212 or 213, t1/t2 is 0.67, or t1/t3 is 0.67 respectively, i.e. a relationship of t2>t1 and t1<t3 is established.

Next, an example of how to use the dilator 1 will be explained.

First, the object is punctured with an introducer needle (not illustrated) to form a hole. Subsequently, a guide wire (not illustrated) is inserted into a through-hole of the introducer needle, and then the introducer needle is drawn out.

Next, the proximal end of the guide wire is inserted into the through-hole 11h of the dilator 1, the dilator 1 is pushed and advanced into the hole site (hereinafter, also referred to as "punctured portion") of the object, and the distal end portion 112 and the tapered portion 111 are inserted into the hole in this order. Subsequently, the tapered portion 111 is further pushed and advanced while rotating the shaft 11, to expand the hole. Incidentally, when the tapered portion 111 expands the hole, it is preferable that the rotation and the pushing of the shaft 11 are combined by manipulating the base portion 31.

As described above, the dilator 1 has the aforementioned configuration, and therefore, when expanding the hole of the object, inroad of the spirally-arranged protruding portion 211 of the tapered portion 111 into a living tissue can be alleviated because the height t1 of the spirally-arranged protruding portion 211 is smaller than the heights t2 and t3 of the spirally-arranged protruding portions 212 and 213. Thereby, the hole of the object can be easily and securely expanded by combining screw adoption for the spirally-arranged protruding portion 21, due to the rotation of the shaft 11, with the pushing of the shaft 11.

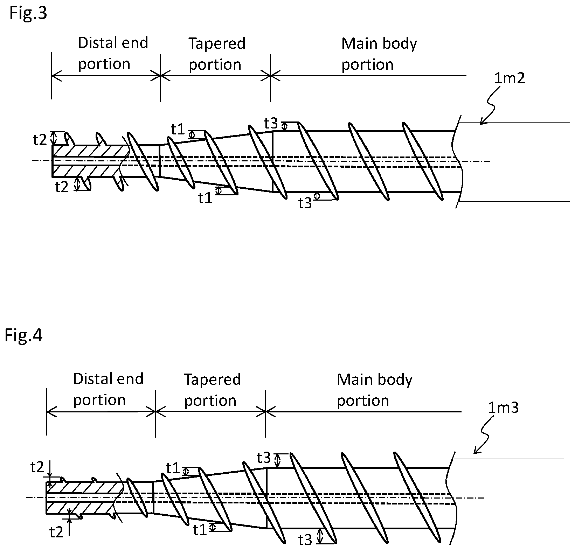

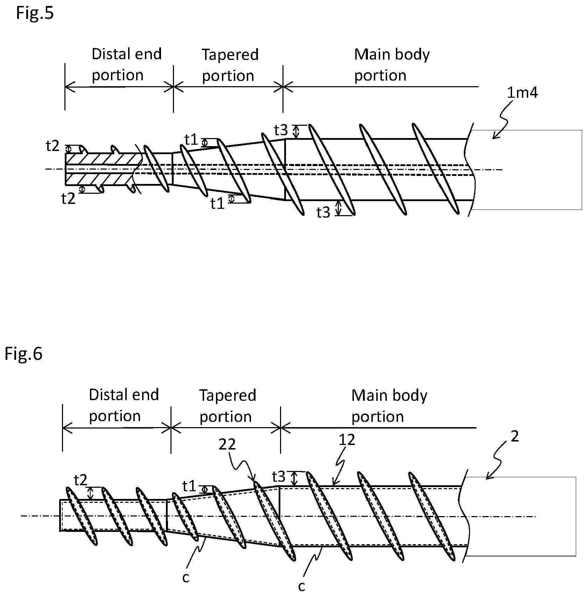

Incidentally, in the dilator 1, the height t1 of the spirally-arranged protruding portion 211 formed on the tapered portion 111 may be smaller than any one of the heights t2 and t3 of the spirally-arranged protruding portions 212 and 213 formed on the distal end portion 112 or the main body 113. For example, the dilator 1 may be a dilator 1m1 with t2>t1>t3 (see FIG. 2), a dilator 1m2 with t2>t1=t3 (see FIG. 3), a dilator 1m3 with t2<t1<t3 (see FIG. 4), and a dilator 1m4 with t2=t1<t3 (see FIG. 5).

### [Second Embodiment]

FIG. 6 is a schematic enlarged side view of a main part illustrating the second embodiment of the present invention. As illustrated in FIG. 6, a dilator 2 is roughly composed of a shaft 12, a spirally-arranged protruding portion 22, and the base portion 31 (not illustrated). The dilator 2 is different from the dilator in the first embodiment in that the dilator 2 includes the shaft 12 and the spirally-arranged protruding portion 22. Incidentally, the same parts as those in the first embodiment are given the same reference numerals, and explanation of the same parts is omitted. In addition, since the configurations in the shaft 12 and the spirally-arranged protruding portion 22 excluding a coating portion c are the same as in the shaft 11 and the spirally-arranged protruding portion 21 respectively in the first embodiment, explanation of the same configurations is omitted.

Each of the shaft 12 and the spirally-arranged protruding portion 22 has the coating portion c in the outer surface side part thereof. Specifically, this coating portion c is a coating film formed in a topmost surface of the shaft 12 and the spirally-arranged protruding portion 22. The coating c is formed, for example, for the purpose of improving a slidability, preventing living tissue from biting into the shaft or spirally arranged protruding portion, and the like. A thickness of the coating portion c may be e.g. 0.1 to 300 µm. Incidentally, a height of the spirally-arranged protruding portion 22 in the present embodiment means a height including the thickness of the coating portion c.

As the material for forming the coating portion c, for example, a biocompatible resin material such as a polyamide resin and a fluororesin, a hydrophilic coating material, or the like can be adopted.

As a method for forming the coating portion c, for example, a known method such as a dipping method and a spray method can be adopted.

### [Third Embodiment]

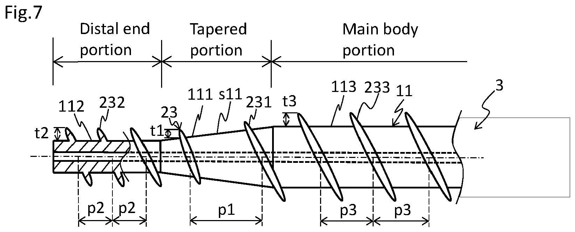

FIG. 7 is a partially cut-out schematic enlarged side view of a main part illustrating the third embodiment of the present invention. As illustrated in FIG. 7, a dilator 3 is roughly composed of the shaft 11, a spirally-arranged protruding portion 23, and the base portion 31 (not illustrated). In the dilator 3, the spirally-arranged protruding portion 23 is different from the spirally-arranged protruding portion in the first embodiment. Incidentally, the same parts as those in the first embodiment are given the same reference numerals, and explanation of the same parts is omitted. In addition, since the configurations excluding the shape in the spirally-arranged protruding portion 23 are the same as those in the spirally-arranged protruding portion 21 in the first embodiment, explanation of the same configurations is omitted.

The spirally-arranged protruding portion 23 is formed on each of outer peripheral surfaces of the tapered portion, and the distal end portion and/or the main body, and has gaps between adjacent sections along the axial direction of the shaft. The spirally-arranged protruding portion formed on the tapered portion has a height smaller than a height of the spirally-arranged protruding portion formed on the distal end portion and/or the main body. Additionally, in the spirally-arranged protruding portion 23, a pitch of the spirally-arranged protruding portion formed on the outer peripheral surface of the tapered portion is larger than a pitch of the spirally-arranged protruding portion formed on the outer peripheral surface of the shaft excluding the tapered portion.

In the present embodiment, the spirally-arranged protruding portion 23 is formed on the outer peripheral surface s11 of the distal end portion 112, the tapered portion 111 and the main body 113, and is formed such that a spirally-arranged protruding portion 231 formed on the tapered portion 111 has a height t1 smaller than heights t2 and t3 of spirally-arranged protruding portions 232 and 233 formed on the distal end portion 112 and the main body 113, and a pitch p1 of the spirally-arranged protruding portion 231 formed on the outer peripheral surface s11 of the tapered portion 111 is larger than pitches p2 and p3 of the spirally-arranged protruding portions 232 and 233 formed on the outer peripheral surface s11 of the shaft 11 excluding the tapered portion 111 (on the outer peripheral surface s11 of the distal end portion 112 and the main body 113).

Ordinarily, as for the pitches of the spirally-arranged protruding portion 23, the pitch p1 in the tapered portion 111 is 0.25 to 5 mm, the pitch p2 in the distal end portion 112 is 0.2 to 4 mm, and the pitch p3 in the main body 113 is 0.2 to 4 mm. Preferably, a ratio of the pitch p1 of the spirally-arranged protruding portion 231 to the pitch p2 or p3 of the spirally-arranged protruding portion 232 or 233 (p1/p2, or p1/p3) is larger than 1 and smaller than or equal to 25.

In the present embodiment, as for the pitches of the spirally-arranged protruding portion 23, the pitch p1 is 2 mm, the pitch p2 is 1.5 mm, and the pitch p3 is 1.5 mm. As for the ratio of the pitch p1 of the spirally-arranged protruding portion 231 to the pitch p2 or p3 of the spirally-arranged protruding portion 232 or 233, p1/p2 is 1.3, or p1/p3 is 1.3 respectively.

As described above, the dilator 3 is formed such that the pitch p1 of the spirally-arranged protruding portion 231 is larger than the pitches p2 and p3 of the spirally-arranged protruding portions 232 and 233. Thus, when the tapered portion 111 expands a hole of an object, a screw action is weakened because the pitch p1 of the spirally-arranged protruding portion 231 is larger, and therefore damage to a living tissue on an inner surface of the hole can be prevented.

### [Fourth Embodiment]

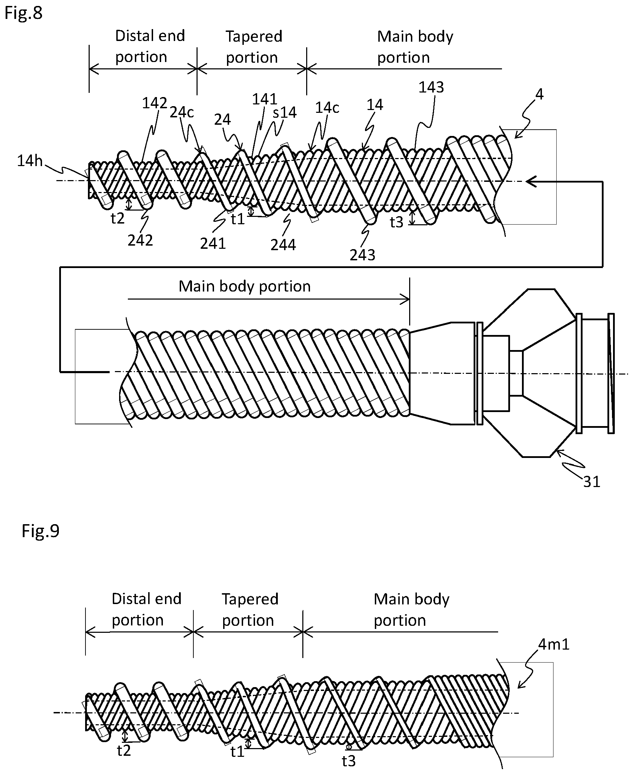

FIG. 8 is a schematic side view illustrating the whole of the fourth embodiment of the present invention. As illustrated in FIG. 8, a dilator 4 is roughly composed of a shaft 14, a spirally-arranged protruding portion 24, and the base portion 31. In the dilator 4, the shaft 14 and the spirally-arranged protruding portion 24 are different from those in the first embodiment. Incidentally, the same parts as those in the first embodiment are given the same reference numerals, and explanation of the same parts is omitted. In addition, since the configurations excluding the shape in the shaft 14 and the spirally-arranged protruding portion 24 are the same as those in the shaft 11 and the spirally-arranged protruding portion 21 respectively in the first embodiment, explanation of the same configurations is omitted.

The shaft 14 is a hollow member having a through-hole 14h. This shaft 14 has a tapered portion 141, a distal end portion 142, and a main body 143. The tapered portion 141 is a part where a outer diameter at a distal end of the tapered portion is smaller than at a proximal end of the tapered portion. The distal end portion 142 is a part having a proximal end positioned on the distal end of the tapered portion 141 and extending toward the axis-directional distal end side of the tapered portion 141. The main body 143 is a part having a distal end positioned on the proximal end of the tapered portion 141 and extending toward the axis-directional proximal end side of the tapered portion 141.

The shaft 14 is composed of a first coil body 14c obtained by winding a wire around an axis of the shaft 14. This first coil body 14c can be formed e.g. by winding one wire around the shaft 14 such that adjacent wires are in close contact with each other along the axial direction of the shaft 14 and a shape of an outer peripheral surface s14 matches with the shapes of the aforementioned distal end portion 142, tapered portion 141, and main body 143. Incidentally, a space formed inside the first coil body 14c (region inside the dashed line in FIG. 8) corresponds to the through-hole 14h.

The spirally-arranged protruding portion 24 is formed on the respective outer peripheral surfaces of the tapered portion, and the distal end portion and/or the main body, and has gaps between adjacent sections along the axial direction of the shaft. The spirally-arranged protruding portion formed on the tapered portion has a height smaller than heights of the spirally-arranged protruding portions formed on the distal end portion and/or the main body.

The spirally-arranged protruding portion 24 is composed of a second coil body 24c obtained by winding the wire around the outer peripheral surface s14 of the shaft 14. Specifically, this spirally-arranged protruding portion 24 can be formed e.g. in such a way that one wire or a plurality of independent wires having a diameter corresponding to respective heights t1, t2, and t3 of spirally-arranged protruding portions 241, 242, and 243 are wound around the outer peripheral surface s14 of the shaft 14 such that the diameter of the wire of the second coil body 24c wound around the outer peripheral surface s14 of the tapered portion 141 is smaller than the diameter of the wire of the second coil body 24c wound around the outer peripheral surface s14 of the shaft 14 excluding the tapered portion 141, and gaps 244 is formed between adjacent sections along the axial direction of the shaft 14.

When the second coil body 24c is composed of one wire, this wire can be obtained e.g. by adjusting the diameter of the wire of the second coil body 24c wound around the outer peripheral surface s14 of the tapered portion 141 so as to be smaller than the diameter of the wire of the second coil body 24c wound around the outer peripheral surface s14 of the shaft 14 excluding the tapered portion 141. As such an adjustment method, e.g. a method of joining a plurality of wire rods having different diameters in series to form one wire, a method of polishing a part of a wire rod (e.g. a part constituting the spirally-arranged protruding portion 241) for use as a material for the wire of the second coil body, a method of overlaying by coating on the other parts (e.g. parts constituting the spirally-arranged protruding portions 242 and 243), or the like can be adopted. Incidentally, examples of a material for use in the above coating include the same materials as cited as the materials for the aforementioned coating c (see FIG. 6), and the like.

On the other hand, when the second coil body 24c is composed of a plurality of independent wires, the second coil body 24c can be formed e.g. by arranging a wire having a diameter corresponding to the height t1 of the spirally-arranged protruding portion 241 on the outer peripheral surface s14 of the tapered portion 141 and arranging wires having diameters corresponding to the heights t2 and t3 of the spirally-arranged protruding portions 242 and 243 on the outer peripheral surface s14 of the distal end portion 142 and the main body 143.

In the fourth embodiment, three wires having diameters corresponding to the heights t1, t2, and t3 (t2>t1, and t1<t3) of the spirally-arranged protruding portions 241, 242, and 243 are joined in series to obtain one wire, and each part having each diameter in this wire is positioned on the outer peripheral surface s14 of the tapered portion 141, the distal end portion 142, and the main body 143 respectively.

The diameter of the wire constituting the first coil body 14c is ordinarily 0.1 to 0.5 mm. The diameter and a diameter ratio of the one wire or the plurality of wires constituting the second coil body 24c can be set to e.g. the same values as the heights t1, t2, and t3 of the spirally-arranged protruding portions 211, 212, and 213 and as the ratio of the height t1 of the spirally-arranged protruding portion 211 to the height t2 or t3 of the spirally-arranged protruding portion 212 or 213 respectively, explained in the first embodiment. In the present embodiment, the wire of the first coil body 14c has a diameter of 0.21 mm. The second coil body 24c is composed of one wire, and the diameters of the wire are 0.3 mm in the distal end portion, 0.2 mm in the tapered portion, and 0.3 mm in the main body.

Examples of the material for the wire constituting the first coil body 14c and the second coil body 24c include, for example, the same materials as the materials constituting the distal end portion 112, the tapered portion 111, and the main body 113, and the materials constituting the spirally-arranged protruding portion 21, each of which has been explained in the dilator 1,and the like.

As a method for joining the first coil body 14c and the second coil body 24c, for example, a method of brazing the end portions of them with a solder material, a welding method, an adhering method using an adhesive, an adhering method by welding with a coating film, or the like can be adopted.

Incidentally, when the shaft 14 is composed of the first coil body 14c and the spirally-arranged protruding portion 24 is composed of the second coil body 24c, it is preferable that the winding direction of the wire in the first coil body 14c and the winding direction of the wire in the second coil body 24c are opposite to each other, i.e. among the first coil body 14c and the second coil body 24c, one is S-twisted and the other is Z-twisted. Thereby, when rotating the shaft 14, directions of the axial forces applied to the wires of the first coil body 14c and the second coil body 24c can be made opposite to each other, separation between the adjacent wires in the first coil body 14c can be prevented to suppress decrease in torquability and pushability.

As described above, in the dilator 4, the shaft 14 is composed of the first coil body 14c and the spirally-arranged protruding portion 24 is composed of the second coil body 24c, so that flexibility and torquability can be improved in the shaft 14 and the spirally-arranged protruding portion 24 respectively, and the flexibility and torquability can be further improved by their synergistic action. In addition, since the spirally-arranged protruding portion 24 is composed of the second coil body 24c in the dilator 4, the heights t1 to t3 of the spirally-arranged protruding portions 241 to 243 can be easily and simply adjusted depending on the diameter of the wire for use.

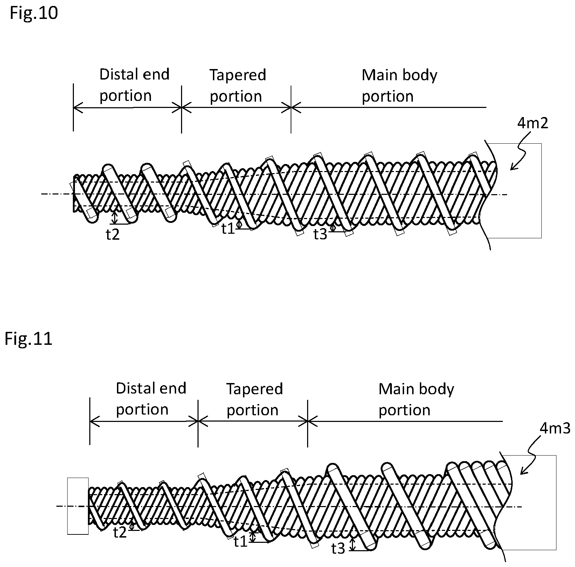

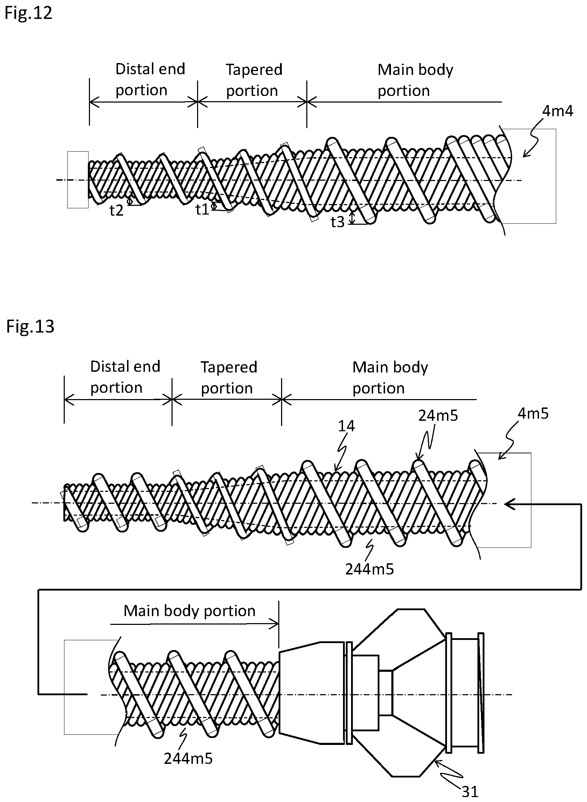

Incidentally, in the dilator 4, the height t1 of the spirally-arranged protruding portion 241 formed on the tapered portion 141 may be smaller than either the height t2 or t3 of the spirally-arranged protruding portions 242 or 243 formed on the distal end portion 142 or the main body 143. For example, the dilator 4 may be a dilator 4m1 with t2>t1>t3 (see FIG. 9), a dilator 4m2 with t2>t1=t3 (see FIG. 10), a dilator 4m3 with t2<t1<t3 (see FIG. 11), and a dilator 4m4 with t2=t1<t3 (see FIG. 12). In addition, the dilator 4 may be a dilator 4m5 in which a second coil body 24m5 has gaps 244m5 between the adjacent sections along the axial direction of the first coil body 14c (shaft 14) up to a proximal end of the first coil body 14c (see FIG. 13).

### [Fifth Embodiment]

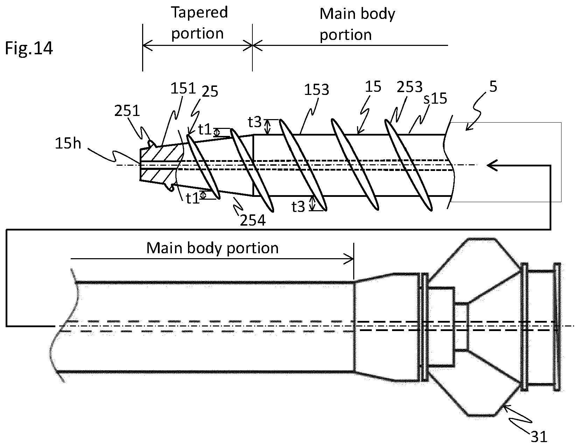

FIG. 14 is a partially cut-out schematic side view illustrating the whole of the fifth embodiment of the present invention. As illustrated in FIG. 14, a dilator 5 is roughly composed of a shaft 15, a spirally-arranged protruding portion 25, and the base portion 31. In the dilator 5, the shaft 15 and the spirally-arranged protruding portion 25 are different from those in the first embodiment. Incidentally, the same parts as those in the first embodiment are given the same reference numerals, and explanation of the same parts is omitted. In addition, since the configurations excluding the shape in the shaft 15 and the spirally-arranged protruding portion 25 are the same as those in the shaft 11 and the spirally-arranged protruding portion 21 respectively in the first embodiment, explanation of the same configurations is omitted.

The shaft 15 is a member having a hollow through-hole 15h. The shaft 15 according to the present embodiment does not have any distal end portion (e.g. see FIG. 1) but has a tapered portion 151 and a main body 153. The spirally-arranged protruding portion 25 is formed on each outer peripheral surface s15 of the tapered portion 151 and the main body 153, and has gaps 254 between adjacent sections along an axial direction of the shaft 15. The spirally-arranged protruding portion 25 is formed such that a height t1 of a spirally-arranged protruding portion 251 formed on the tapered portion 151 is smaller than a height t3 of a spirally-arranged protruding portion 253 formed on the main body 153. Incidentally, since configurations of the tapered portion 151 and main body 153, and the spirally-arranged protruding portions 251 and 253 are the same as the configurations of the tapered portion 111 and main body 113, and the spirally-arranged protruding portions 211 and 213 respectively in the first embodiment, explanation of the same configurations is omitted.

As described above, the dilator 5 has the aforementioned configuration, and therefore, when expanding a hole of an object, inroad of the spirally-arranged protruding portion 251 of the tapered portion 151 into a living tissue can be alleviated because the height t1 of the spirally-arranged protruding portion 251 is smaller than the height t3 of the spirally-arranged protruding portion 253. Thereby, the hole of the object can be easily and securely expanded by combining screw adoption for the spirally-arranged protruding portion 251 due to the rotation of the shaft 15, with the pushing of the shaft 15.

### [Sixth Embodiment]

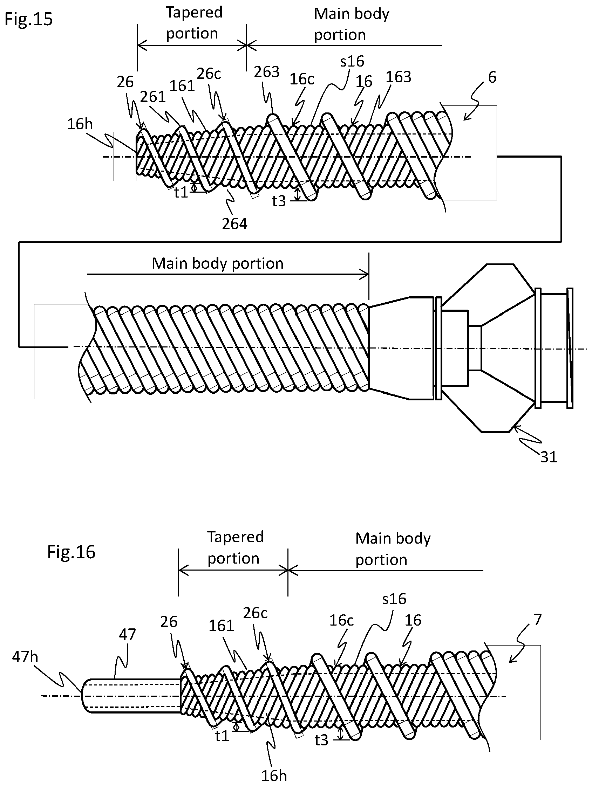

FIG. 15 is a schematic side view illustrating the whole of the sixth embodiment of the present invention. As illustrated in FIG. 15, a dilator 6 is roughly composed of a shaft 16, a spirally-arranged protruding portion 26, and the base portion 31. In the dilator 6, the shaft 16 and the spirally-arranged protruding portion 26 are different from those in the fourth embodiment. Incidentally, the same parts as those in the first embodiment are given the same reference numerals, and explanation of the same parts is omitted.

The shaft 16 is a member having a hollow through-hole 16h. This shaft 16 is composed of a first coil body 16c obtained by winding a wire around an axis of the shaft 16, and has the tapered portion 161 and the main body 163. The spirally-arranged protruding portion 26 is formed on each outer peripheral surface s16 of the tapered portion 161 and the main body 163, and has gaps 264 between adjacent sections along an axial direction of the shaft 16. The spirally-arranged protruding portion 26 is composed of a second coil body 26c obtained by winding a wire around the outer peripheral surface s16 of the shaft 16, and formed such that a diameter of the wire of the second coil body 26c wound around the outer peripheral surface s16 of the tapered portion 161 is smaller than a diameter of the wire of the second coil body 26c wound around the outer peripheral surface s16 of the main body 163. Incidentally, since configurations of the tapered portion 161 and the main body 163, and configurations of spirally-arranged protruding portions 261 and 263 are the same as the configurations of the tapered portion 141 and the main body 143, and the configurations of the spirally-arranged protruding portions 241 and 243 respectively in the fourth embodiment, explanation of the same configurations is omitted.

As described above, in the dilator 6, the shaft 16 is composed of the first coil body 16c and the spirally-arranged protruding portion 26 is composed of the second coil body 26c, so that flexibility and torquability can be improved in the shaft 16 and the spirally-arranged protruding portion 26 respectively, and flexibility and torquability can be further improved by their synergistic action. In addition, since the spirally-arranged protruding portion 26 is composed of the second coil body 26c in the dilator 6, heights t1 and t3 of the spirally-arranged protruding portions 261 and 263 can be easily and simply adjusted depending on the diameter of the wire for use.

### [Seventh Embodiment]

FIG. 16 is a schematic enlarged side view of a main part illustrating the seventh embodiment of the present invention. As illustrated in FIG. 16, a dilator 7 is roughly composed of the shaft 16, the spirally-arranged protruding portion 26, a distal tip 47, and the base portion 31 (not illustrated). The dilator 7 is different from the dilator in the sixth embodiment in that the dilator 7 includes the distal tip 47. Incidentally, the same parts as those in the first and the sixth embodiments are given the same reference numerals, and explanation of the same parts is omitted.

The distal tip 47 is a part having a proximal end positioned on the distal end of the shaft 16 and extending toward the axis-directional distal end side of the tapered portion 161. The distal tip 47 has a surface flatter than the outer peripheral surface s16 (uneven surface due to the wire) of the first coil body 16c, and includes an substantially hollow cylindrical through-hole 47h communicating with the through-hole 16h of the shaft 16. Incidentally, in the present embodiment, an inner diameter of the through-hole 47h of the distal tip 47 is 0.7 mm, and inner diameters of the through-hole 16h of the shaft 16 are 0.7 mm on the distal end, and 1.5 mm on the proximal end.

The distal tip 47 can be formed e.g. by flattening the surfaces of the distal end portions of the first coil body 16c and the second coil body 26c using a solder material such as a silver-tin solder and a gold-tin solder, or the like.

As described above, since the dilator 7 includes the distal tip 47, insertability of the shaft 16 into the hole bored by an introducer needle can be improved, and the procedure can be smoothly advanced.

Note that the present invention are not limited to the configurations of the aforementioned embodiments, but is indicated by claims, the disclosed embodiments are intended to include all modifications within the meaning and scope equivalent to those in claims.

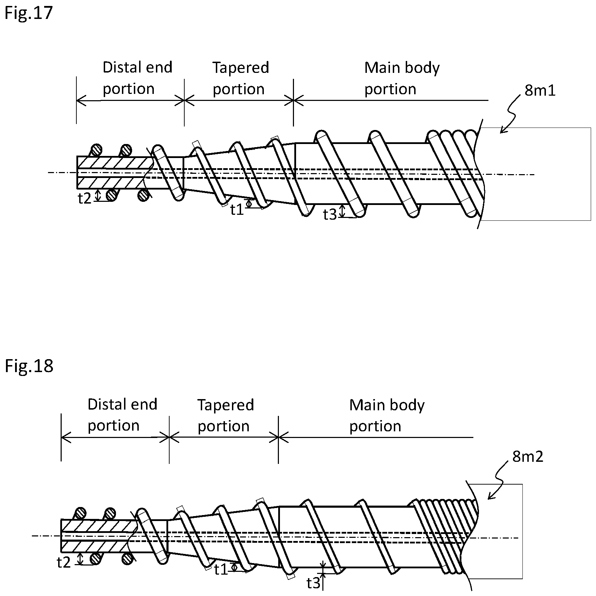

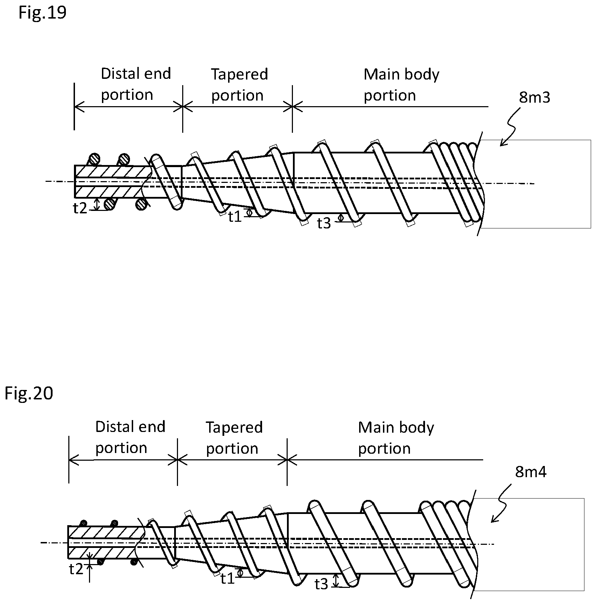

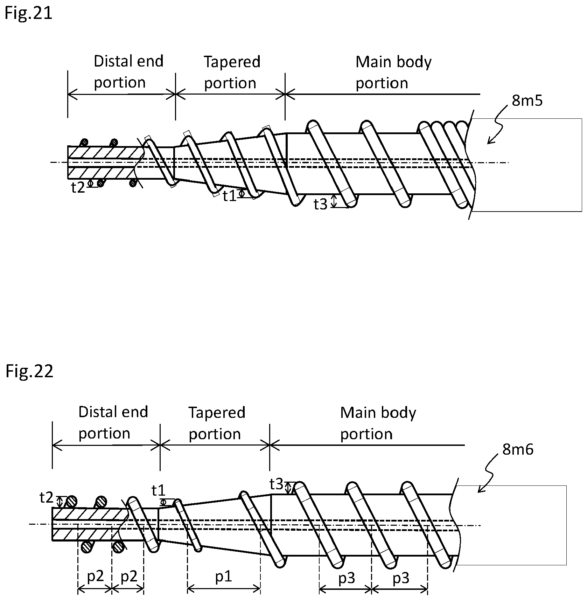

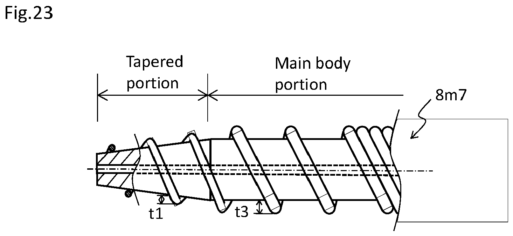

For example, the dilators 1 to 3 in which the shaft 11 and the spirally-arranged protruding portions 21 and 23 are integrally formed have been explained in the aforementioned first to third embodiments, and the dilators 4, 6, and 7 in which each of the shafts 14 and 16 and the spirally-arranged protruding portions 24 and 26 is composed of the coil body have been explained in the fourth, sixth, and seventh embodiments, but the dilator may be a dilator in which only the parts of the shaft are integrally formed and the spirally-arranged protruding portion is composed of the coil body (second coil body), e.g. a dilator 8m1 with t2>t1 and t1<t3 (see FIG. 17), a dilator 8m2 with t2>t1>t3 (see FIG. 18), a dilator 8m3 with t2>t1=t3 (see FIG. 19), a dilator 8m4 with t2<t1<t3 (see FIG. 20), a dilator 8m5 with t2=t1<t3 (see FIG. 21), a dilator 8m6 with t2>t1, t1<t3, p1>p2 and p3 (see FIG. 22), or a dilator 8m7 with t1<t3 (see FIG. 23).

In addition, the dilator 2 including the coating portion c has been explained in the aforementioned second embodiment, the dilator 3 in which the spirally-arranged protruding portions 23 having different pitches are mixed has been explained in the third embodiment, and the dilator 7 including the distal tip 47 has been explained in the seventh embodiment, but one or two or more of these configurations may be appropriately incorporated in the dilator as long as the effect of the disclosed embodiments is not impaired. For example, the distal tip 47 cited in the seventh embodiment (see FIG. 16) can be adopted for any dilator according to the present invention including the aforementioned first to sixth embodiments.

In addition, in the aforementioned first to third and fifth embodiments, the dilators 1 to 3 and 5 in which the spirally-arranged protruding portions 21 to 23 and 25 are formed from the distal end to the middle part in the axial direction on the outer peripheral surfaces of the shafts 11, 12, and 15 respectively have been explained, but the dilator may be a dilator in which the spirally-arranged protruding portion is formed throughout the axial direction on the outer peripheral surface of the shaft.

In addition, in the aforementioned sixth and seventh embodiments, the dilators 6 and 7 in which the spirally-arranged protruding portion 26 (second coil body 26c) has gaps between adjacent sections from the distal end to the middle part in the axial direction on the outer peripheral surface of the shaft 16, and adjacent sections are in contact with each other from the middle part to the proximal end have been explained, but the dilator may be a dilator in which the spirally-arranged protruding portion (second coil body) has gaps between adjacent sections throughout the axial direction (from the distal end to the proximal end) on the outer peripheral surface of the shaft.

### REFERENCE SIGNS LIST

- 1 to 7, 1m1 to 1m4, 4m1 to 4m5, 8m1 to 8m7: Dilator
- 11, 12, 14 to 16: Shaft
- 111, 141, 151, 161: Tapered portion
- 112, 142: Distal end portion
- 113, 143, 153, 163: Main body
- 14c, 16c: First coil body
- 21 to 26: Spirally-arranged protruding portion
- 24c, 26c: Second coil body
- 214, 234, 244, 254, 264: Gap
- s11, s14 to s16: Outer peripheral surface
- t1, t2, t3: Height of spirally-arranged protruding portion
- p1, p2, p3: Pitch of spirally-arranged protruding portion

## Claims

1. A dilator comprising a hollow shaft and a spirally-arranged protruding portion, wherein
the shaft comprises either
a tapered portion having an outer diameter that is smaller at a distal end of the tapered portion than at a proximal end of the tapered portion,
a distal end portion having a proximal end positioned on a distal end of the tapered portion and extending toward an axis-directional distal end side of the tapered portion, and
a main body having a distal end positioned on a proximal end of the tapered portion and extending toward an axis-directional proximal end side of the tapered portion, or
the tapered portion and the main body without the distal end portion, and
in a case where the shaft does not have the distal end portion, the spirally-arranged protruding portion is formed on each of outer peripheral surfaces of the tapered portion and the main body and has gaps between adjacent sections along an axial direction of the shaft, and
the spirally-arranged protruding portion formed on the tapered portion has a height smaller than a height of the spirally-arranged protruding portion formed on the main body, and
in a case where the shaft has the distal end portion, the spirally-arranged protruding portion is formed on each of outer peripheral surfaces of the tapered portion, and the distal end portion and/or the main body and has gaps between adjacent sections along the axial direction of the shaft, and
the spirally-arranged protruding portion formed on the tapered portion has a height smaller than heights of the spirally-arranged protruding portions formed on the distal end portion and/or the main body.

2. The dilator according to claim 1, wherein the shaft is composed of a first coil body comprising a wire wound around an axis of the shaft.

3. The dilator according to claim 1 or 2, wherein the spirally-arranged protruding portion is composed of a second coil body comprising a wire wound around on the outer peripheral surface of the shaft.

4. The dilator according to 1, wherein
the shaft is composed of the first coil body comprising a wire wound around the axis of the shaft, and the spirally-arranged protruding portion is composed of the second coil body comprising a wire wound around on the outer peripheral surface of the shaft, and
a winding direction of the wire on the first coil body and a winding direction of the wire on the second coil body are opposite to each other.

5. The dilator according to claim 3 or 4, wherein
the wire of the second coil body wound around the outer peripheral surface of the tapered portion has a diameter smaller than a diameter of the wire of the second coil body wound around on the outer peripheral surface of the shaft excluding the tapered portion.

6. The dilator according to any one of claims 1 to 5, wherein
a pitch of the spirally-arranged protruding portion formed on the outer peripheral surface of the tapered portion is larger than a pitch of the spirally-arranged protruding portion formed on the outer peripheral surface of the shaft excluding the tapered portion.
